(19) **Europäisches Patentamt**

**European Patent Office**

**Office européen des brevets**

(11) **EP 4 546 351 A1**

(12) **EUROPEAN PATENT APPLICATION**

(43) Date of publication:
**30.04.2025 Bulletin 2025/18**

(21) Application number: **23306874.1**

(22) Date of filing: **27.10.2023**

(51) International Patent Classification (IPC):
**G16B 20/20** (2019.01)     **C12Q 1/6869** (2018.01)
**G16B 30/00** (2019.01)

(52) Cooperative Patent Classification (CPC):
**G16B 30/00; G16B 20/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **SORBONNE UNIVERSITE**
  **75006 Paris (FR)**
- **ASSISTANCE PUBLIQUE - HÔPITAUX DE PARIS**
  **75012 Paris 12 (FR)**

- **Institut National de la Santé et de la Recherche Médicale**
  **75013 Paris (FR)**

(72) Inventors:
- **VILLARD, Eric**
  **Peris (FR)**
- **PERRET, Claire**
  **Paris (FR)**

(74) Representative: **Cabinet Beau de Loménie**
  **158, rue de l'Université**
  **75340 Paris Cedex 07 (FR)**

(54) **METHOD FOR DETECTING RARE MUTATIONS IN GENOMIC DNAS OF A POPULATION**

(57)     The invention relates to a computer implemented method for detecting at least one mutation in the genome of a member of a population and to a method for detecting at least one mutation in the genome of a member of a population.

**EP 4 546 351 A1**

**Description**

**Background of the invention**

The advent of massively parallel DNA sequencing, such as next-generation sequencing (NGS), has ushered in a new era of genomic exploration by making simultaneous genotyping of hundreds of billions of base-pairs possible at small fraction of the time and cost of traditional Sanger methods. Because these technologies digitally tabulate the sequence of many individual DNA fragments, unlike conventional techniques which simply report the average genotype of an aggregate collection of molecules, they offer the unique ability to detect minor variants within heterogeneous mixtures.

Deep sequencing, however, has limitations. Although, in theory, DNA subpopulations of any size should be detectable when deep sequencing a sufficient number of molecules, a practical limit of detection is imposed by errors introduced during sequencing and/or during sample preparation. From 0.001% to 1% of bases are incorrectly identified, depending on the sequencing method. This background level of artefactual heterogeneity establishes a limit below which the presence of true variants are detected.

A variety of improvements at the level of biochemistry and data processing have been developed to improve sequencing accuracy. The ability to resolve subpopulations below 0.1%, however, has remained elusive. Although several groups have attempted to increase sensitivity of sequencing, several limitations remain.

The most efficient technology to perform genetic diagnosis of a disease, such as Dilated cardiomyopathy (DCM), is targeted sequencing on a large panel of genes, however it remains expensive while molecular diagnosis prescription increases. In this context, combinatorial pool-DNA sequencing (Pool-Seq) could be a time- and cost-effective approach. In addition, it has been reported that two-dimensional Pool-Seq allows for the identification of rare variants with determination of carrier DNA, after decoding of the pools. The advantage of the pooling is that not all sequences have to be determined separately, but that the pools allow for rapid identification of mutations in a population. This facilitates the screening of large or complex populations in particular.

However, it is still desirable to develop an approach for reducing or eliminating artefactual mutations arising from DNA damage, PCR errors, and/or sequencing errors; allowing rare variants to be detected in members of a population.

In the present invention, we developed a new method based on two-dimensional Pool-Seq strategy (allowing sequencing large numbers of DNAs from patients) and based on a specific data analysis strategy for detecting of mutations. The method of the invention has a better efficiency, and has a cost and time advantage over the methods disclosed in the prior art, such as Next Generation Sequencing (NGS) based simplex DNA capture methods with minimal loss of sensitivity or specificity for rare variants detection.

Therefore, the method of the invention is particularly advantageous for detecting mutations in routine genetic diagnosis in rare diseases.

**Summary of the invention**

In a first aspect, the present invention relates to a computer implemented method for detecting at least one mutation in the genome of a member of a population $M_1,..., M_X$, the method comprising the steps of:

- obtaining pools $P_1,...,P_N$ of nucleotide sequences such that:

   ◦ each pool $P_n$ containing nucleotide sequences of a DNA product $A_n$ obtained from genomic DNA samples $S_1,...,S_K$ of K population members;
   ◦ each genomic DNA sample $S_k$ used to obtain a DNA product $A_n$ of a pool $P_n$ is also used to obtain only one DNA product $A_p$ of a pool $P_p$, the pools $P_n$ and $P_p$ being so-called concordant pools,
   ◦ each pair of concordant pools $P_n$ and $P_p$ shares only one originating genomic DNA sample $S_{k*}$ from which the DNA products $A_n$ and $A_p$ are obtained,

- identifying mutation(s) by clustering said nucleotide sequences of DNA products and aligning the clustered nucleotide sequences with a reference sequence,
- attributing a detection reliability score to each of the mutation(s) identified in the DNA products; and
- detecting a mutation in a genome if:

   ◦ the mutation is identified in the nucleotide sequences of DNA products only in a pair of concordant pools ; and
   ◦ the detection reliability score associated with said mutation exceeds a reference value.

In a particular embodiment, the various steps of the computer implemented method are determined by computer program instructions or are implemented by a silicon chip which comprises transistors adapted to constitute logic gates of a non-programmable wired logic.

Accordingly, the invention also relates to a computer program on a data carrier, this program being capable of being implemented in a controller computer, this program comprising instructions adapted to implement the steps of a detecting method as described above.

This program can use any programming language, and be in the form of source code, object code, or code intermediate between source code and object code, such as in a partially compiled form, or in any other desirable form.

The invention also relates to a computer-readable information carrier containing instructions for a computer program as mentioned above. The information carrier may be any entity or device capable of storing the program. For example, the medium may comprise a storage medium, such as a ROM, a flash-type non-volatile memory or a magnetic recording medium, such as a hard disk. On the other hand, the information carrier can be a transmissible medium such as an electrical or optical signal, which can be conveyed via an electrical or optical cable, by radio or by other means. In particular, the program according to the invention can be downloaded from an Internet-type network. Alternatively, the information carrier may be an integrated circuit in which the program is incorporated, the circuit being adapted to execute or to be used in the execution of the process in question.

In another aspect, the present invention relates to a method for detecting at least one mutation in the genome of a member of a population $M_1,..., M_X$, comprising the steps of:

a) providing genomic DNA samples $S_1,...,S_K$ of each population members;

b) pooling the genomic DNA samples obtained in step a) thereby obtaining a DNA product in each pool $P_n$, wherein each pools $P_1,...,P_N$ comprises K genomic DNA samples of K population members, wherein each of the K genomic DNA samples is present in only two pools $P_n$ and $P_p$, the pools $P_n$ and $P_p$ being so-called concordant pools, and wherein each pair of concordant pools $P_n$ and $P_p$ shares only one common genomic DNA sample, ;

c) optionally, fragmenting the DNA product in each pool $P_n$, thereby obtaining a fragmented DNA product in each pool $P_n$ ;

d) optionally, in addition to step c), capturing a target sequence of interest in the fragmented DNA product in each pool $P_n$, thereby obtaining a captured DNA product in each pool $P_n$ ;

e) optionally, amplifying the DNA product of step b), the fragmented DNA product of step c) or the captured DNA product of step d) in each pool $P_n$, thereby obtaining an amplified DNA product in each pool $P_n$;

f) determining the nucleotide sequence of the DNA product of step b), of the fragmented DNA product of step c) or of the amplified DNA product of step e) in each pool $P_n$, thereby obtaining pools $P_1,...,P_N$ of nucleotide sequences ;

g) detecting a mutation by implementing the computer implemented method according to the invention;

h) optionally, confirming a mutation that is detected in step g) by determining the sequence of a target sequence that is suspected to carry said mutation from a genomic DNA sample of the member that is suspected to carry said mutation.

## Brief description of the drawings

Further features and advantages of the present invention will become apparent from the description given below, with reference to the attached drawings, which illustrate nonlimiting examples. On the figures:

Figure 1 shows a flowchart of the main steps of a detection method according to a particular embodiment of the invention.

Figure 2 illustrates concordant pools and non-concordant pools in the sense of the present disclosure.

Figure 3 is a chart presenting the percentage of unique mutations in concordant pools relative to all unique mutations (in concordant pools and non-concordant pools) as a function of quantile of 0.1 log(mQUAL) increment.

Figure 4 shows distribution of the Freebayes mQUAL logarithmic value for all mutations identified once in 2 concordant pools, expected to be true mutations (empty bars) or 2 non-concordant pools, assumed to be false positives (hatched bars).

Figure 5 shows random set of true or false mutations replication status after Sanger sequencing in concordant pools. Mutations detected in 2 concordant pools were randomly selected (n=56) among those with a mQUAL value ranging [0.1-50] and plotted for the best-of-2-pool QUAL value (mQUAL) on the abscissa and the best-of-2-pool mutant allele coverage (mDPAlt) in the ordinate. Empty circles (on the right): True mutations, validated by Sanger method. Full circles (on the left): False mutations, not validated by Sanger method.

Figure 6 shows the hardware architecture of a detection system according to a particular embodiment of the invention.

## Detailed description of specific embodiments

## A) Computer implemented method

In a first aspect, the invention relates to a computer implemented method for detecting at least one mutation in the genome of a member of a population $M_1,..., M_X$, the method comprising the steps of:

- obtaining pools $P_1,...,P_N$ of nucleotide sequences such that:

  ○ each pool $P_n$ containing nucleotide sequences of a DNA product $A_n$ obtained from genomic DNA samples $S_1,...,S_K$ of K population members;
  ○ each genomic DNA sample $S_k$ used to obtain a DNA product $A_n$ of a pool $P_n$ is also used to obtain only one DNA product $A_p$ of a pool $P_p$, the pools $P_n$ and $P_p$ being so-called concordant pools,
  ○ each concordant pools $P_n$ and $P_p$ share only one originating genomic DNA sample $S_{k*}$ from which the DNA products $A_n$ and $A_p$ are obtained,

- identifying mutation(s) by clustering said nucleotide sequences of DNA products and aligning the clustered nucleotide sequences with a reference sequence,
- attributing a detection reliability score to each of the mutation(s) identified in the DNA products; and
- detecting a mutation in a genome if:

  ○ the mutation is identified in the nucleotide sequences of DNA products only in a pair of concordant pools ; and
  ○ the detection reliability score associated with said mutation exceeds a reference value.

In the context of the invention, the term "mutation" means an alteration in the nucleic acid sequence of a genome. The mutation may be an insertion, a deletion or a substitution. For example, the mutation may be a SNP (single-nucleotide polymorphism).

## Obtaining pools $P_1,...,P_N$ of nucleotide sequences

The computer implemented method comprises a step of obtaining pools $P_1,...,P_N$ of nucleotide sequences.
In some embodiments, the pools $P_1,...,P_N$ of nucleotide sequences are obtained by implementing the following steps:

  a) providing genomic DNA samples $S_1,...,S_K$ of each population members;
  b) pooling the genomic DNA samples obtained in step a) thereby obtaining a DNA product in each pool $P_n$, wherein each pools $P_1,...,P_N$ comprises K genomic DNA samples of step a) of K population members, wherein each of the K genomic DNA samples is present in only two pools $P_n$ and $P_p$, the pools $P_n$ and $P_p$ being so-called concordant pools, and wherein each concordant pools $P_n$ and $P_p$ shares only one common genomic DNA sample, ;
  c) optionally, fragmenting the DNA product in each pool $P_n$, thereby obtaining a fragmented DNA product in each pool $P_n$ ;
  d) optionally, in addition to step c), capturing a target sequence of interest in the fragmented DNA product in each pool $P_n$, thereby obtaining a captured DNA product in each pool $P_n$ ;
  e) optionally, amplifying the DNA product of step b), the fragmented DNA product of step c) or the captured DNA product of step d) in each pool $P_n$, thereby obtaining an amplified DNA product in each pool $P_n$ ;
  f) determining the nucleotide sequence of the DNA product of step b), of the fragmented DNA product of step c) or of the amplified DNA product of step e) in each pool $P_n$, thereby obtaining pools $P_1,...,P_N$ of nucleotide sequences.

### Step a)

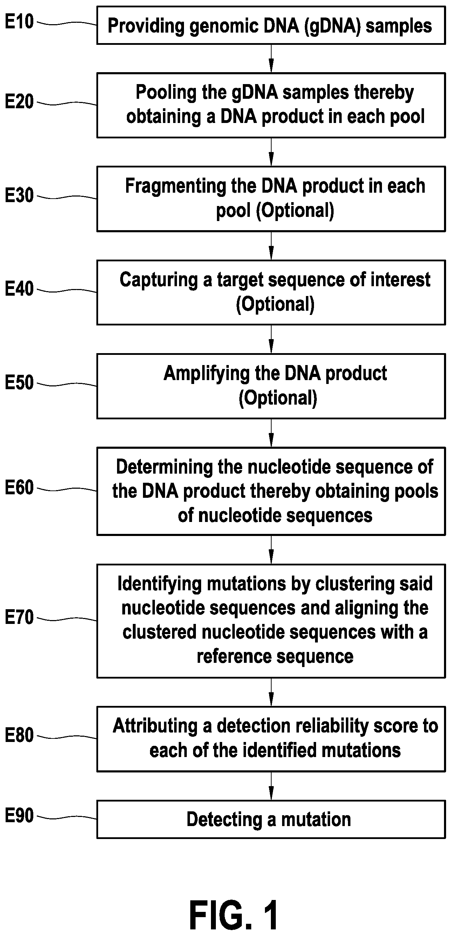

Step a) consists in providing genomic DNA samples $S_1,...,S_K$ of each population members (Step E10 in Figure 1). Genomic DNA samples are obtained by isolating genomic DNA of each member of the population to provide for the genomic DNA samples of each member in the population. The isolation of DNA is generally achieved using common methods in the art such as the collection of tissue from a member of the population and DNA extraction.

### Step b)

Step b) consists in pooling the genomic DNA samples obtained in step a) thereby obtaining a DNA product in each pool $P_n$, wherein each pools $P_1,...,P_N$ comprises K genomic DNA samples of step a) of K population members, wherein each of the K genomic DNA samples is present in only two pools $P_n$ and $P_p$, the pools $P_n$ and $P_p$ being so-called concordant pools, and wherein each concordant pools $P_n$ and $P_p$ shares only one common genomic DNA sample (Step E20 in Figure 1).

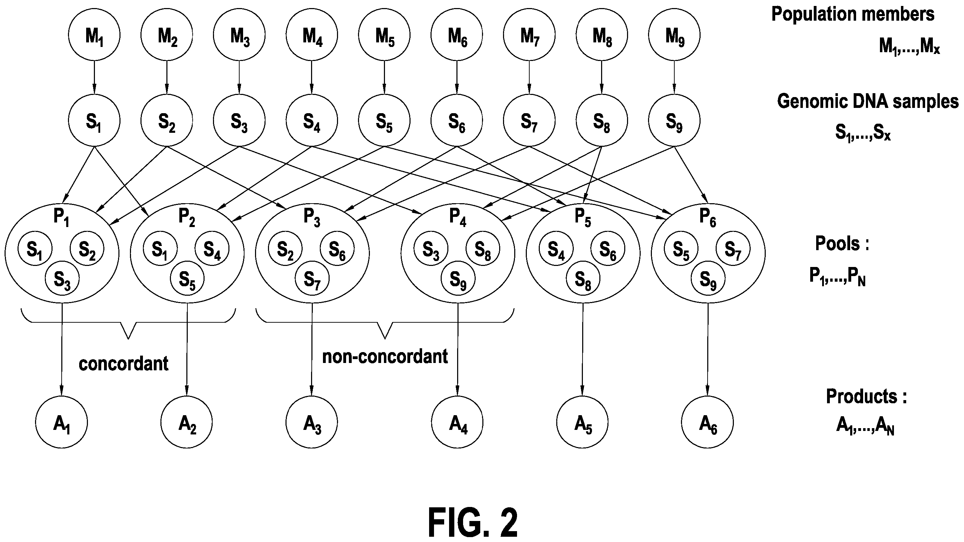

As illustrated on Figure 2; two concordant pools $P_n$ and $P_p$ share only one originating genomic DNA sample $S_{k*}$ from which the DNA products $A_n$ and $A_p$ are obtained. In other words, each concordant pools $P_n$ and $P_p$ do not share two or more originating genomic DNA samples $(S_{k*})$ from which the DNA products $(A_{k*,n})$ $(A_{k*,p})$ are obtained.

By definition, two pools $P_n$ and $P_p$ that do not meet said definition of concordant pools are said to be non-concordant pools.

The number of population members is not particularly limited. It may be 10 members or more, 20 members or more, 50 members or more, 100 members or more, 150 members or more, 200 members or more, 250 members or more, 300 members or more, for example 350 members or more.

The number of genomic DNA samples in each pool, i.e. "K", is not particularly limited and will depend on the number of population members. In some embodiments, K is an integer comprised from 2 to 500, such as from 2 to 250, such as from 2 to 100, such as from 2 to 50, such as from 2 to 40, from 2 to 30, from 2 to 20, for example from 2 to 10. For example, but not limited to, for a population of 100 to 500 members, such as from 300 to 400 members, K may be an integer from 4 to 15, such as from 5 to 10.

The number of pools, i.e. "$P_N$", is not particularly limited and will depend on the number of population members and on the value of "K". For practical reasons, the number of pools is preferably at most 96, which corresponds the number of well of a 96-well plate.

Pooling may serve to normalize the amount of each genomic DNAs to provide for a more equal representation in the pools for sequencing. In a preferred embodiment, the pooling is achieved using equal amounts of genomic DNA for each member.

According to an embodiment, we have X the number of members in the population, and we select K=√X the number of genomic DNA sample in each pool, and N=2·√X the number of pools. Considering a given population of members $M_1,...,M_X$, this embodiment has the advantage of minimizing the number of sequencing required to detect a mutation in the genome of a member of the population $M_1,..., M_X$.

In the simplified illustration of Figure 2, we consider a population of 9 members $M_1, ..., M_9$ (X=9), 3 genomic DNA samples in each pool (i.e. K=3), and 6 pools (N=6).

The pool $P_1$ contains (otherwise comprises only) nucleotide sequences of a DNA product $A_1$ obtained only from genomic DNA samples $S_1$ of member $M_1$, genomic DNA samples $S_2$ of member $M_2$, and genomic DNA samples $S_3$ of member $M_3$.

The pool $P_2$ contains (otherwise comprises only) nucleotide sequences of a DNA product $A_2$ obtained only from genomic DNA samples $S_1$ of member $M_1$, genomic DNA samples $S_4$ of member $M_4$, and genomic DNA samples $S_5$ of member $M_5$.

The genomic DNA sample $S_1$ is used to obtain only two fragmented DNA products $A_1$ and $A_2$. Pools $P_1$ and $P_2$ are concordant because they share (or both comprise) one and only one genomic DNA sample, in this case $S_1$.

Conversely, the pools $P_3$ and $P_4$ are non-concordant because they do not share any genomic DNA sample in common.

Within the scope of the claimed invention, it could be envisaged other embodiments for forming the pools, and in particular for the selection of parameters X, K, and N.

*Optional step c)*

Optional step c) consists in fragmenting the DNA product in each pool $P_n$, thereby obtaining a fragmented DNA product in each pool $P_n$ (Step E30 in Figure 1). The fragmentation of the DNA products is generally achieved using common methods in the art such as physical techniques, i.e. shearing, sonication, or other random fragmentation methods.

The fragmentation step may be necessary for implementing next-generation sequencing (NGS), such as Illumina sequencing and SOLiD sequencing.

In some embodiments, sequencing adaptors may be ligated to the DNA fragments. The sequencing adaptor includes at least a region for annealing to a bead, a sequencing primer region and/or a PCR primer region.

*Optional step d)*

Optional step d) consists in, preferably in addition to step c), capturing a target sequence of interest in the fragmented DNA product in each pool $P_n$, thereby obtaining a captured DNA product in each pool $P_n$ (Step E40 in Figure 1). The capture of the sequence of interest is generally achieved using common methods in the art such as using a probe, such as a probe attached to a solid support, for example beads on which nucleic acid probes are attached, or using a probe linked to a Biotin group that is retained on a streptavidin beads for purification of the target sequence.

The capture step may serve to isolate a target sequence of interest to focus the sequencing on a target sequence of interest, such as a target sequence of interest that may comprise a mutation associated with a disease.

*Optional step e)*

Optional step e) consists in amplifying the DNA product of step b), the fragmented DNA product of step c) or the captured DNA product of step d) in each pool $P_n$, thereby obtaining an amplified DNA product in each pool $P_n$ (Step E50 in Figure 1). The amplification of a DNA product is generally achieved using common methods in the art such as PCR using pair of primers.

*Step f)*

Step f) consists in determining the nucleotide sequence of the DNA product of step b), of the fragmented DNA product of step c) or of the amplified DNA product of step e) in each pool $P_n$, thereby obtaining pools $P_1,...,P_N$ of nucleotide sequences (Step E60 in Figure 1). The sequencing of a DNA product is generally achieved using common methods in the art such as Next-Generation Sequencing (NGS). In some embodiments, the NGS is chosen from Massively Parallel Signature Sequencing (MPSS), Illumina sequencing, Pyrosequencing, SOLiD sequencing, Single molecule real time (RNAP) sequencing, Single molecule SMRT sequencing.

In some embodiments, steps c) to f) correspond to a Next-Generation Sequencing (NGS) process, such as Massively Parallel Signature Sequencing (MPSS) process, Illumina sequencing process, Pyrosequencing, SOLiD sequencing process, Single molecule real time (RNAP) sequencing process or Single molecule SMRT sequencing process.

*Identifying mutation(s)*

The computer implemented method comprises a step E70 of identifying mutation(s) by clustering said nucleotide sequences of DNA products and aligning the clustered nucleotide sequences with a reference sequence.

"Aligning and clusturing" mean the comparison of two or more nucleotide sequences based on the presence of short or long stretches of identical or similar nucleotides. Several methods for alignment of nucleotide sequences are known in the art.

In some embodiments, the method uses the Freebayes genetic mutation caller (e.g. Freebayes v1.1.0) to identify mutations by comparing nucleotide sequences of DNA products to a reference sequence.

The reference sequence may correspond to a wild-type sequence. For example, if the method is implemented to perform genetic diagnosis of a disease, the reference sequence corresponds to a sequence of a healthy subject for said disease.

The reference sequence may also correspond to a sequence built from the alignment, with the reads presenting the most frequent allele at each position.

The majority of the sequences are identical to the reference sequence (not mutated). The mutations comprised in a genomic DNA (true mutations) and mutations introduced during sequencing and/or during sample preparation (false mutations) are both identified at this step.

*Attributing a detection reliability score to each of the identified mutation(s)*

The computer implemented method comprises a step E80 of attributing a detection reliability score to each of the mutation(s) identified in the DNA products.

In some embodiments, the method uses the Freebayes genetic mutation caller (e.g. Freebayes v1.1.0) to attributing a detection reliability score to each of the mutation(s) identified in the DNA products.

As known by the person skilled in the art, Freebayes attributes (step E80) a "quality score" (QUAL score) representing a scaled probability of the mutation being true, the higher the QUAL score, the more confident Freebayes is in the accuracy of the mutation call.

The skilled person understands that a true mutation in a target sequence of a given member $S_k$ has a high probability of being detected with a high quality score in concordant pools containing sequences obtained from genomic DNA samples of this member.

Conversely, we understand that the false positives, i.e. the mutations due to sequencing errors, have high probability of being detected with a low quality score. In addition, the probability of detecting a false mutation is identically distributed across pairs of pools, whether or not those are concordant.

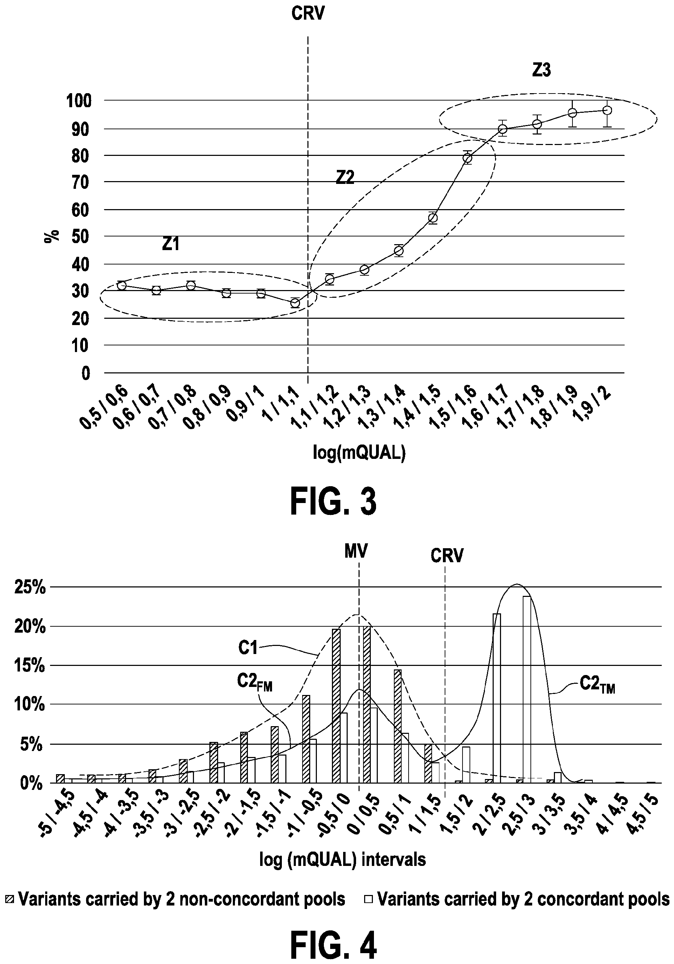

This is perfectly illustrated by the chart of Figure 3 which we describe hereinafter. This chart relates to unique mutations (i.e. mutations that are detected only once in the DNAs included in the pools). Specifically, it presents the percentage of unique mutations in concordant pools relative to all unique mutations (in concordant and non-concordant pools) as a function of quantile of 0.1 log(mQUAL) increment, +/- margin of error (at 5%). mQUAL is the higher of the two QUAL values : either the QUAL from $P_p$ or $P_n$ attributed to the same mutation.

Generally speaking, the curve has a first zone Z1 consisting of a first plateau, a second zone Z2 with a rising (or increasing) curve, and a third zone Z3 with a second plateau.

The zone Z1 corresponds to mutations associated with low-quality scores which are probably false mutations. It should be reminded that the probability of detecting a false mutation, due to a sequencing error, is similar whether a pair of pools is concordant or not. The percentage of these false mutations in concordant pairs of pools compared with all the mutations is therefore relatively stable, and corresponds to the number of concordant pairs of pools compared with the total number of pair of pools.

In the Z2 zone, the curve shows a significant increase in the percentage of unique mutations in concordant pools as the quality of mutations, measured by log(mQUAL), increases. This is due to the fact that high quality mutations are more likely to result from a real mutation in a target sequence rather than a mutation due to sequencing error.

Above certain quality, the curve reaches a second plateau (zone Z3) where the percentage of unique mutations in concordant pairs of pools stabilizes. This indicates that a majority of true mutations have been identified, and that additional quality does not add many more matching mutations.

Consequently, the quality score at the boundary between the Z1 and Z2 zones may be used as a coarse reference value CRV for discriminating:

- true mutations, considered to be those associated with a detection reliability score higher than said coarse reference value; and
- false mutations (e.g. mutations due to sequencing errors), considered to be those associated with a detection reliability score lower than said coarse reference value.

This coarse reference value may be used to define a range in which a refined reference value must be precisely determined (see below).

Several methods may be used to determine the coarse reference value.

*Methods for determining the coarse reference value*

a/ The coarse reference value CRV may be detected when the percentage of unique mutations in concordant pools relative to all unique mutations as a function of quantiles of log(mQUAL) increment increases more than a predetermined threshold for two consecutive quantiles.

b/ The coarse reference value CRV may be detected graphically when the curve representing the percentage of unique mutations in concordant pools relative to all unique mutations as a function of log(mQUAL) shows an inflection.

c/ The coarse reference value CRV may be detected mathematically by modelling said curve by a function and by examining the zeros, minima, maxima of the first derivative and/or the second derivative of said function.

In the example of Figure 3, the coarse reference value approximately corresponds to a value of mQual such that log(mQual) = 1.1. Therefore *mQual* = $10^{1.1}$ = 12,6

d/ The coarse reference value can also be detected from a graph of the type shown in Figure 4.

Figure 4 represents with hatched bars mutations carried by two non-concordant pools and with empty bars mutations carried by two concordant pools.

In fact, the percentage of false mutations due to sequencing errors detected in two pools can be considered a random variable, fitting a curve annotated C1.

Similarly, false mutations due to sequencing errors may also occur in two concordant pools. This explains the fact that the probability distribution of this variable for two concordant pools is represented by a curve annotated $C2_{FM}$ with a similar shape to the curve C1 (except for the scaling). It follows that the false mutations (due to sequencing errors) in concordant pools have a high probability of being detected with low-quality scores.

On the contrary, the detection reliability score (i.e. the quality score) of a true mutation in two concordant pools is much higher, than the detection reliability score (i.e. the quality score) of a false mutation in two concordant pools. In other words, the true mutations in the concordant pools have a high probability of being detected with a high quality score.

The coarse reference value CRV may then be determined at the intersection, of the C1 and C2 curves which allows discriminating between the false mutations (due to sequencing errors and associated with low-quality scores) and the true mutations in the target sequences (associated with high-quality scores).

In conclusion, the mutations detected in concordant pools include: i) false mutations (due to sequencing errors) with a high probability of being detected with low quality scores - curve $C2_{fm}$, and ii) true mutations in the target sequence with a high probability of being detected with high quality scores - curve $C2_{tm}$. The coarse reference value CRV is used to discriminate between these false mutations and true mutations.

*Detecting a mutation in a genome*

The computer implemented method comprises a step E90 of detecting a mutation in a genome if:

- ◦ the mutation is identified in the nucleotide sequences of DNA products only in concordant pools ; and
- ◦ the detection reliability score associated with said mutation exceeds a reference value.

The reference value allows discriminating between true mutations and false mutations.

The coarse reference value CRV may be used to obtain a RRV refined reference value that may be determined using Sanger Sequencing

In one embodiment, a refined reference value is determined within said range by sequencing, using Sanger Sequencing, the genomic DNAs of the members from which a mutation have been identified in concordant pairs of pools within the range. The Sanger sequencing allows confirming whether or not said identified mutation is a true mutation or a false mutation.

Sanger Sequencing has been performed on a set of samples for which the mQual score is comprised in a range centred around the coarse reference value (12.6).

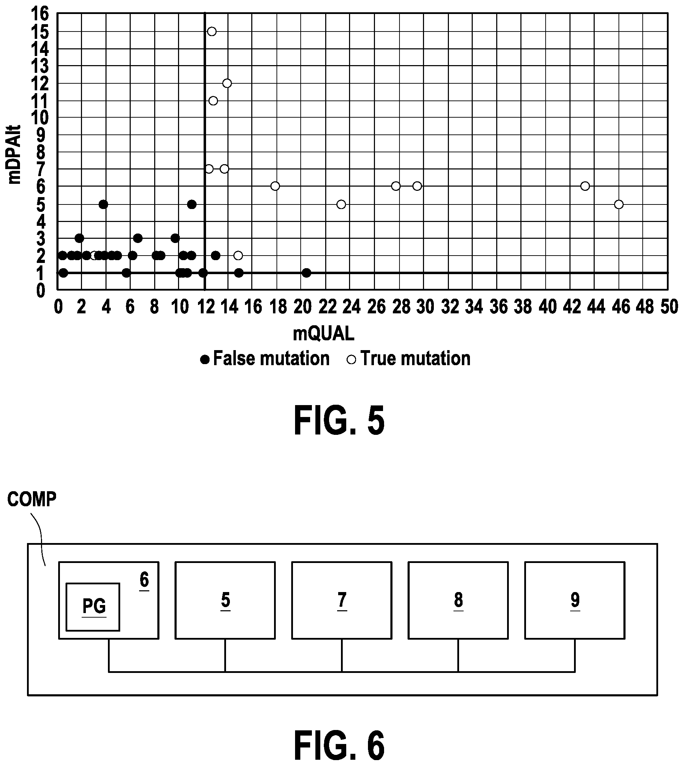

Figure 5 represents in true mutations with empty circles (mainly on the right of the bar), and mutations due to sequencing errors with full circles (mainly on the left of the bar).

The refined reference value RRV may be determined as to minimize the sum of the mDPAtl of true mutations with a mQUAL inferior to the refined reference value and mDPAlt of false mutations with a mQUAL superior to the refined reference value. This ensures reliable detection, i.e. minimising the number of detection errors. mDPAlt is the highest number of observation of the mutation in $P_n$ or $P_p$ alignment.

In the example of Figure 5; the refined reference value RRV is determined equal to 12.

In the context of the claimed invention, the reference value used for detecting a mutation is a target sequence (step E90) may be either the above mentioned coarse or refined reference values.

In one embodiment, a further condition for detecting said mutation in said genome is that the mutation is observed at least a predetermined number of times, in at least one pool of the pair of the $P_n$ and $P_p$ concordant pools.

This supplemental condition contributes to improving the reliability of the proposed detection method.

As illustrated by Figure 5, this condition can be verified using the mdPAlt score (Y axis). In Figure 5, this predetermined number is 2 (illustrated as strictly superior to 1). Other predetermined numbers could also be used.

Figure 6 shows the hardware architecture of a detection system, e.g, a computer COMP, according to a particular embodiment of the invention. In the embodiment described here, the detection system has the hardware architecture of a computer. It comprises a processor 5, a ROM 6, a RAM 7, a rewritable non-volatile memory 8 and communication means 9.

The ROM 6 constitutes a recording medium in the sense of the invention. It contains a PG computer program with instructions for executing the steps of a detection method in accordance with the invention, when this program is executed by processor 5.

**B) Method for detecting at least one mutation in a genome**

The present invention also relates to a method for detecting at least one mutation in the genome of a member of a population $M_1,..., M_X$, comprising the steps of:

  a) providing genomic DNA samples $S_1,...,S_K$ of each population members;
  b) pooling the genomic DNA samples obtained in step a) thereby obtaining a DNA product in each pool $P_n$, wherein each pools $P_1,...,P_N$ comprises K genomic DNA samples of K population members, wherein each of the K genomic DNA samples is present in only two pools $P_n$ and $P_p$, the pools $P_n$ and $P_p$ being so-called concordant pools, and wherein each pair of concordant pools $P_n$ and $P_p$ shares only one common genomic DNA sample ;
  c) optionally, fragmenting the DNA product in each pool $P_n$, thereby obtaining a fragmented DNA product in each pool $P_n$ ;
  d) optionally, in addition to step c), capturing a target sequence of interest in the fragmented DNA product in each pool $P_n$, thereby obtaining a captured DNA product in each pool $P_n$ ;
  e) optionally, amplifying the DNA product of step b), the fragmented DNA product of step c) or the captured DNA product of step d) in each pool $P_n$, thereby obtaining an amplified DNA product in each pool $P_n$;
  f) determining the nucleotide sequence of the DNA product of step b), of the fragmented DNA product of step c) or of the amplified DNA product of step e) in each pool $P_n$, thereby obtaining pools $P_1,...,P_N$ of nucleotide sequences ;

g) detecting a mutation by implementing the computer implemented method according to the invention;

h) optionally, confirming a mutation that is detected in step g) by determining the sequence of a target sequence that is suspected to carry said mutation from a genomic DNA sample of the member that is suspected to carry said mutation.

Steps a) to g) are detailed above in section "A) Computer implemented method".

Optional step h) consists in confirming a mutation that is detected in step g) by determining the sequence of a target sequence that is suspected to carry said mutation from a genomic DNA sample of the member that is suspected to carry said mutation. In some embodiment, a detected mutation may be confirmed by sequencing, using Sanger Sequencing, the genomic DNAs of the members from which a mutation have been detected. The Sanger Sequencing allows confirming whether or not said detected mutation is a true mutation or a false mutation.

In some embodiments, the member has or is susceptible to develop a genetic disorder, such as a dilated cardiomyopathy (DCM). According to the invention, a genetic disorder is a disorder having genetic causes, caused by a mutation in a single gene (monogenic) or multiple genes (polygenic).

### EXAMPLES

In the following example, the term "variant" means "mutation" according to the invention.

Dilated cardiomyopathy (DCM) is a heart disease characterized by left ventricular dilatation and systolic dysfunction[1]. It is an important cause of systolic heart failure, and is the first indication for heart transplantation[1]. Pathogenic variants, essentially private to each patient, are identified in about 30% of cases, in more than 50 genes, indicating strong genetic heterogeneity[1]. Then, the most efficient technology to perform genetic diagnosis of DCM is targeted sequencing on a large panel of genes, however it remains expensive while molecular diagnosis prescription increases. In this context, combinatorial pool-DNA sequencing (Pool-Seq) could be a time- and cost-effective approach[2]. In addition, it has been reported that two-dimensional Pool-Seq allows for the identification of rare variants with determination of carrier DNA, after decoding of the pools[3].

In the present report, we performed such a two-dimensional Pool-Seq strategy to sequence 384 DNAs from DCM patients and developed a specific data analysis method for identification of unique variants. We reported the resulting variant atlas in a large panel of 109 genes previously associated with cardiomyopathies and cardiac arrhythmias. In addition, we evaluated the efficiency of the strategy and demonstrated a clear cost and time advantage of Pool-Seq over NGS based simplex DNA capture with minimal loss of sensitivity or specificity for rare variants detection, showing that Pool-Seq can be used for routine genetic diagnosis in rare diseases.

### 1. Materials and Methods

### Population inclusion

The participants included in the present study were selected from 4 independent studies. They had a diagnosis of idiopathic DCM according to conventional criteria characterized by enlarged left ventricle diameter and low ejection fraction ($\leq 50\%$) in the absence of recognized causal factors, such as coronary artery disease[1,2].

A summary of patient characteristics is given below and the details of the 4 studies are in section "cohort description".

| Patient characteristics * | |
|---|---|
| Gender (M / F) | 311 / 68 |
| Origin | France : 320 (83%) Europe : 54 (14%) |
| Family history for DCM | 25% |
| Age of Diagnostic | 46,3 ± 8,7 |
| EF (%) | 28,5 ± 6,8 |
| LVD (mm) | 70 ± 6,7 |
| Heart Transplantation | Yes 176 / No 191 |
| *The number of patients can be less than 384 because of missing data | |

All participants gave written informed consent. All samples were collected in accordance with the Helsinki declaration and study protocols were approved by the ethics committees "CPP comité de protection des personnes dans la recherche biomédicale, Faculty hospital Pitié-Salptrière, Paris" (ref 66-01 and 63-05).

**DNA pooling Library preparation, Capture and Sequencing**

DNAs extracted from peripheral leukocytes of 384 patients with cardiomyopathy was included. DNAs were pooled into 96 8-DNA pools using a Biomek3000 automated system (Beckman Coulter Inc, Brea, CA, USA) to reduce technical variability. Each DNA was included twice and in 2 different pools, so that each DNA appears in a single matched pair of pools. Importantly, DNA concentration of each DNA was carefully measured in triplicate with the Quant-iT PicoGreen dsDNA reagent (Thermofisher Scientific, Waltham, MA, USA) using automated system (Biomek 3000), to control for the equal contribution of each DNA to each pool. Each 8-DNA pool was considered as it if was simplex DNA sample for library preparation, capture and sequencing steps: 8-DNA pool concentration was measured with the Quant-iT Picogreen dsDNA reagent, and indexed library of each pool was prepared from 1 μg of pooled DNA using the TruSeq DNA Sample Prep V2 kit with 12 different index, according to the manufacturer's instructions (Illumina, San Diego, CA, USA).

The present study was a sub-analyse of a broader sequencing effort targeting a total of 1 792 071 pb in 2909 DNA regions. For the here reported analysis, we focused on exons and 20 nucleotides intron boundaries of 109 so-called cardiomyopathy and cardiac arrhythmia genes [3], representing 465 385 bp in 1902 targeted regions. Targeted exons were captured on 3 mixed libraries, with Nimblegen SeqCap EZ choice library (Roche Nimblegen, Madison, WI, USA). The resulting 32 post-captured libraries were sequenced on an 8-lanes flowcell of a HiSeq2500 Illumina sequencer with TruSeq v3 reagent 2x100bp (Illumina, San Diego, CA, USA).

**Alignment and variant calling**

The raw data were aligned on human genomic sequence hg37 using BWA v0.6.2[4]. Variant calling was performed using Bayesian genetic variant detector, Freebayes v1.1.0, with the following parameters settings adapted for 8 DNA-pools data analysis: --pooled-discrete;-ploidy 16; --min-alternate-fraction 0.01; --min-coverage 1; --use-best-n-alleles 5; --min-alternate-total=1. For the --use-best-n-allele parameter, we set 5 to evaluate only the 5 best SNP alleles among the 16 possible ones and limit memory usage [5]. The quality criteria for variant calling was defined from the distribution of Freebayes QUAL score among all variants observed in concordant pools (representing true positives) compared to non-concordant pools (representing false positives) as detailed in results section.

**Pool decoding and carrier DNA identification.**

Since the known pathogenic variants in cardiomyopathy are essentially unique to one DCM case and DCM is a rare disease (1/2500), we expect only a single occurrence of any pathogenic variant in the 384 samples. Therefore, we select variants called in a single DNA carrier *i.e.* in a single concordant pair of pools (unique variants). To take into account the normal presence of false positives in the pool data, we defined unique variants after quality selection on variants initially carried by up to 6 pools. Indeed, because of massive sequencing enabled by NGS, false positive events occurring by chance are high (estimated to 25,000 with technical base error rate 0.001 (Q30) and total aligned bases 925,125,991) even though the probability of such an event is very low (P=0.000028).

**Power validation of combinatorial pool method**

To assess the variant detection power of our Pool-Seq protocol, we compare the variant calling in 50 DNAs and 57 genes (exonic sequences only) shared with a previously published study using standard simplex capture NGS protocol[6].

**Annotation of selected variants**

Variants were annotated using Ensembl Variant Effect Predictor (VEP) tool [7] accounting for i) frequency (gnomAD v2 allele count <10 or unknown)[8], ii) consequence and prediction scores on the canonical transcript : CADD ≥ 30 for frameshift and stop, spliceAI ≥ 0.8 for splice variants, functional prediction tools (SIFT ≤ 0.05, PolyPhen2-Hvar ≥ 0.909, FATHMM ≤ -1.5) and combination tools (REVEL ≥ 0.5, MetaLR ≥0.5) for missense variants. We also looked at the pathogenic report in cardiomyopathies or other cardiac diseases, essentially using Clinvar [9]. On the resulting selected variants, we applied the ACMG standard criteria with 5-class classification and retained only the higher 2 classes, pathogenic (class 5), probably pathogenic (class4) [10]. For TTN variants, we selected only those located in the exons with a PSI score greater than 0.9.

The resulting pathogenic and likely pathogenic variants never described are accessible in ClinVar (ID SUB13444431).

**Sanger sequencing**

To refine mQUAL value threshold 54 called variants were analysed by Sanger Sequencing. PCR amplification was performed using BioTaq (Bioline GmbH, Luckenwalde, Germany) with a standard protocol and PCR product was Sanger-sequenced externally by Genewiz Company (Leipzig, Germany) using primers.

## 2. Cohort description

DCM samples for this study came from 4 studies, European Heart Study (EHF), CARDIGENE, PHRC and IDCM.

**CARDIGENE study.** Cases were French patients with a diagnosis of idiopathic DCM (enlarged left ventricle end-diastolic volume/diameter, LVEDD, >140 ml/m2 on ventriculography or >34 mm/m2 on echocardiography and low ejection fraction, EF, ≤40% confirmed over a six-month period, in the absence of causal factors such as coronary artery disease (coronary angiography was mandatory if DCM occurred after 35 years of age) or sustained arterial hypertension, intrinsic valvular disease, documented myocarditis, congenital malformation, and insulin-dependent diabetes. Only apparently sporadic DCM cases without an additional (first-degree) relative with DCM were included. All were of European origin (born in France, from parents born in France or neighbouring countries). Recruitment was performed in ten hospitals from six French regions (Lille, Lyon, Nancy, Nantes, Paris-Ile de France, and Strasbourg) from September 1994 to February 1996.

A total of 171 DCM cases were included (145 men and 26 women, among them 164 had undergone cardiac transplantation). The mean age of patients at diagnosis was $40.6 \pm 9.2$ years, mean left ventricular ejection fraction (LVEF) was $20.9 \pm 7\%$ and mean left ventricular diameter was $75.3 \pm 10.8$ mm. The study was supported by grants from Délégation à la recherche clinique AP-HP (EMUL and PHRC n°AOM95082).

**EUROGENE study (EHF).** All cases were patients of European origin (all born in Europe, from parents and grandparents born in France or neighboring countries) with a diagnosis of idiopathic DCM, i.e. left ventricle end-diastolic volume/diameter >117% of predicted value according to age and body surface area on echocardiography and low ejection fraction (<45%) confirmed over a three-month period, in the absence of causal factors such as coronary artery disease (coronary angiography or coronary CT scan was mandatory if DCM occurred after 35 years of age) or intrinsic valvular disease, documented myocarditis, systemic disease, sustained rapid supraventricular arrhythmia, or congenital malformation. Recruitment was performed in 11 hospitals in seven European countries from September 2000 to February 2005. For this study, 120 DCM cases were included (91 men and 29 women, 8 patients had undergone cardiac transplantation at inclusion). 69 were french patients and 51 european (essentially from Germany and Italy). The mean age of patients at diagnosis was $46.2 \pm 12.8$ years, mean LVEF was $30.1 \pm 11.5\%$ and mean left ventricular diameter was $67 \pm 9.3$ mm. The study was supported by grants from the "Fondation LEDUCQ".

**PHRC study.** DCM cases were French patients of European origin (all born in France, from parents and grandparents born in France or neighboring countries; some patients of Maghreb origin were retrospectively excluded) with a diagnosis of idiopathic DCM enlarged left ventricle end-diastolic volume/diameter >117% of predicted value according to age and body surface area on echocardiography and low ejection fraction (<45%) clinically stable over a three-month period, in the absence of causal factors such as coronary artery disease (coronary angiography or coronary CT scan was mandatory if DCM occurred after 35 years of age) or intrinsic valvular disease, documented myocarditis, or congenital malformation. Only apparently sporadic DCM cases without additional (first degree) relative with DCM were included. Recruitment was performed in eight hospitals in six regions in France (Lille, Lyon, Nantes, Nice, Paris-Ile de France, and Tours) from October 2005 to November 2008. A total of 64 DCM cases were included in the present study (57 men and 7 women, 4 patients had undergone cardiac transplantation at inclusion). The mean age of patients at diagnosis was $50.5 \pm 12.2$ years, mean LVEF was $28.8 \pm 9.6\%$ and mean left ventricular diameter was $70.1 \pm 9.9$ mm. The study was supported by grants from "Programme Hospitalier de Recherche Clinique" (PHRC n°AOM 04141).

**IDCM study.** Patients were mainly of European origin (94%) and recruited in France. The male/female gender ratio was 63:36 and mean age was $47 \pm 18$ years. Most cases were presenting with isolated DCM (n=92; 96%), some were characterized by DCM and skeletal muscular dystrophy or conduction defect (n=3; 3%), or early atrial fibrillation (*n*=1; 1%). 29 DCM cases were included in the present study with following characteristics: mean age of diagnostic was $42.7 \pm 17.8$ years old, mean LVFE was $33.8 \pm 10.9\%$ and mean LVD was $68.6 \pm 8.4\%$.

## 3. Results

**Sequencing metrics**

We designed the experiment to over-cover each haplome in the pools with 25 reads, that is 400 reads (25x16) per target base. The average coverage observed was slightly higher with 496X and despite 25% of the reads being off-target. For 90.4 % of captured bases, coverage was at least 128X corresponding to an average haplome coverage of 8X allowing at least one coverage for each haplome (p=0.049).

**Cost-cutting evaluation**

Pool-Seq uses only 96 libraries and capture reactions to process 384 DNAs. Given the additional time and resources required for accurate DNA quantitation and pooling, we estimate that saving reagents and labor time reduces the cost of the protocol threefold. A similar 4-fold saving is obtained while sequencing since we reached ~500X coverage in average, which is a depth similar to those used in simplex DNAs NGS protocols.

**Quality filtering of unique variants**

Since the known pathogenic variants are essentially private, we selected only variants called in a single DNA concordant pair of pools (unique variant). To maximize true variant identification in pools, we called all variants with at least one mutated read in two concordant pools. Importantly, these variants could be false positives due to sequencing errors, especially in low coverage regions. To isolate true variants from false positives, we take advantage of the Freebayes QUAL score and compared the distribution of the best-of-2-pools QUAL value (mQUAL) in concordant pairs versus in non-concordant pairs, characterizing false positives (**Figure 4**). The variants identified only in concordant pairs display a log(mQUAL)>1,5. This threshold, corresponding to mQUAL>32, might allow to maximize true positive while decreasing calling false-positive variants.

To refine this mQUAL threshold, we amplified by PCR and re-sequenced a set of candidate variants (n=54) called from unique concordant pairs of pools with mQUAL values in the range [0.1 -50]. From Sanger sequencing results, true (n=13) or false-positive (n=41) status was assigned to each variant and each was plotted according to mQUAL and mDPAlt (*ie* the number of reads carrying the alternate allele compare to reference genome) on **Figure 5.** All but one false positive (1/41; 2.4%) are excluded, and all true-positives are retained (13/13) when applying the following thresholds: (i) mQUAL>12 and (ii) mDPAlt>1, suggesting an excellent discriminating potential of the filtering based on mQUAL and mDPAlt.

**Power-to-detect estimate of the pooling strategy**

We compared Pool-Seq unique variant detection rate to a previously published NGS based sequencing of simplex DNA sharing 50 DNAs with our study[4]. In these 50 DNAs, 319 unique variants were identified in simplex. In the pool sequencing results, after applying mQUAL>12 and mDPAlt>1 filters, 305 out of the 319 were identified in the expected concordant pairs. Twelve of the 14 missing SNVs were confirmed as false-negative after Sanger sequencing indicating a false positive rate of 0.63% (2/319) in simplex. Of the 12 false-negative variants in Pool-Seq (12/317; 3.8%), 9 were detected in only 1 of the 2 concordant pools (termed "partial false-negative pool") and 3 not detected at all.

Conversely, of 190 variants found only once in a single pair of concordant pools containing one of the 50 DNAs shared with Haas et al. study, 187 were also present after standard simplex capture NGS (98.9%), in the same DNA. Two were false negatives in simplex as they were confirmed after Sanger sequencing (false-negative rate = 1.1%). The other was a concordant pools false positive (0.5%).

We achieved an almost identical rare variant detection rate with both methods (96% vs. 99%) at similar coverage indicating that the pooling strategy could reduce the sequencing depth and thus the sequencing cost by 4-fold.

**Variants calling in cardiomyopathy patient's DNAs**

*Variants identification*

From the pooled DNAs of 384 patient with standard criteria for DCM (see supporting information), we selected unique variants present in a single pair of pools after applying predefined QUAL and coverage thresholds (mQUAL<12, mDPAlt>1).

We have therefore identified a total of 1596 unique variants with expected 1/16 average allele frequency in exons or intron-exon boundaries in 107 genes for 383 samples. After annotation, 102 variants of interest (ACMG class 4 or 5) were identified in 100 DCM patients and 36 genes. Among them, 22 (21.6%) were missense and 80 (78.4%) were truncating variants (34 frameshift, 11 splice-site and 35 Stop codon). If only the 19 genes with strong evidence for association with DCM were considered, according to ClinGen consortium[5], 95 of this 102 variants are retained. Regarding the genes spectrum, class 4&5 variants are mainly TTN truncation, but also MYH7 missense, FLNC truncation and TNNT2 missense for the most frequent. Regarding the variants spectrum, 49 are already reported and 46 are new ones, absent in gnomAD or ClinVar database.

## 4. Discussion

In the present targeted-NGS strategy, we describe a simple and robust combinatorial 8-DNA pooling strategy to detect unique variants with high sensitivity and reduced cost.

Our objective was to select, in DNA-pools, all unique variants that could cause the disease, without increasing sequencing depth. To be able to detect them in low coverage region, we reduced the minimal mutated base coverage to 1X, with the cost of increasing sequencing error calling. Our original combinatorial pool strategy drastically limits the false-positives rate. Moreover, filtering optimization based on mQUAL and mDPAlt parameters allowed to limit false-positive rate to ~0.5% only.

We also calculated a very good true-positive detection rate of 96.2% in pool, with a mean coverage similar to which generally applied to genetic diagnosis (400X), constituting a strong improvement compare to previously published pool-sequencing[3]. The 12 false-negative (3.8%), may be mainly explained by local low coverage. Indeed, 10 are under-covered compared to an established 15X necessary for simplex sequencing calling[6], indicating they would have been recovered with deeper sequencing, reaching 99.4% sensitivity identical to what calculated from standard simplex capture results reported in the present study. A very accurate quantitation of DNAs before and after pooling is mandatory to avoid allelic drop-out that could increase false negative rate.

We also demonstrated the combinatorial pooling method capacity to identify the DNA carrier in pools for unique variants. Nevertheless, this method can be used exclusively to detect unique variants because the carrier DNA determination can be ambiguous with more than 2 carrier pools. So, it is particularly suitable for genetic diagnosis in disease associated with private variants. Identification of hotspots, or familial variants, occurring more than once in the DNAs included in the pools, or variants validation would need optimization on pools constitution, filtering strategy and/or extra orthogonal validation using sequencing on an independent sample, generating extra costs.

Interestingly, our Pool-seq strategy allows to reduce significantly the cost of genetic diagnosis since the cost of library preparation and target sequence capture were reduced by 3 and the sequencing cost by 4.

Considering only the 19 most confirmed DCM genes[5], they harbour 93% of ACMG class 4 & 5 variants in 25% of the included DNAs. Our variant detection rate is in the lower part of previously reported rates (20 and 35 %)[5], probably due to high number of sporadic cases in our study (75%) as previously reported [7]. We observed 57% of variants being TTN truncating variant (TTNtv), nearly twice more than reported[8]. This TTNtv enrichment could be explained by high rate of carriers with severe phenotype since 56% of them had heart transplantation[9].

The majority of detected variations was in known, high-confident, DCM genes such as TTN, MYH7, FLNC and TNNT2. However, we also identified class 4 & 5 variants in genes mainly associated with other cardiomyopathies such as HCM with MYBPC3tv (n=2) or ARVC with PKP2tv (n=1). This might be related to (i) uncertainty when defining the genes responsible for DCM, or (ii) in difficulties in phenotypic classification of some patients since end-stage phase of some cardiomyopathies may mimic DCM, especially when early conventional phenotypic phase was not recognized[10]

To progress towards personalized medicine and optimal treatment of cardiomyopathies and other genetic diseases, genetic diagnosis is increasingly requested by clinicians. Here we demonstrated that Pool-Sequencing is a cost- and time-effective NGS strategy for the diagnostic identification of rare variants. In addition, our study increases knowledge of the atlas of DCM gene variants.

### Limitations

These results are not accounting for diversity of human genetic backgrounds. Accordingly, refinement of the thresholding strategy with more ancestry-diverse and larger cohorts will be required to validate the selected parameters. CNVs were not searched for since it is a rarely reported cause for cardiomyopathy and they are still not optimally detected by capture-based NGS strategy.

### REFERENCES

1. Pinto, Y. M. et al. Proposal for a revised definition of dilated cardiomyopathy [...]. Eur. Heart J. 37, 1850-1858 (2016).

2. Cao, C. & Sun, X. Combinatorial pooled sequencing: experiment design and decoding. Quant. Biol. 4, 36-46 (2016).

3. Zuzarte, P. C. et al. A Two-Dimensional Pooling Strategy for Rare Variant Detection on Next-Generation Sequencing Platforms. PLoS ONE 9, e93455 (2014).

4. Haas, J. et al. Atlas of the clinical genetics of human dilated cardiomyopathy. Eur. Heart J. 36, 1123-1135 (2015).

5. Jordan, E. et al. Evidence-Based Assessment of Genes in Dilated Cardiomyopathy. Circulation 144, 7-19 (2021).

6. Hartman, P. et al. Next generation sequencing for clinical diagnostics: Five year experience of an academic laboratory. Mol. Genet. Metab. Rep. 19, 100464 (2019).

7. Hershberger, R. E. et al. Genetic evaluation of cardiomyopathy: a clinical practice resource of the American College of Medical Genetics and Genomics (ACMG). Genet. Med. 20, 899-909 (2018).

8. Herman, D. S. et al. Truncations of Titin Causing Dilated Cardiomyopathy. N. Engl. J. Med. 366, 619-628 (2012).

9. Roberts, A. M. et al. Integrated allelic, transcriptional, and phenomic dissection of the cardiac effects of titin truncations in health and disease. Sci. Transl. Med. 7, (2015).

10. Stolfo, D., Collini, V. & Sinagra, G. Advanced Heart Failure in Special Population: Cardiomyopathies and Myocarditis. Heart Failures Clinics 17 (4); 661-672 (2021).

**Claims**

1. A computer implemented method for detecting at least one mutation in the genome of a member of a population $M_1,...,M_X$, the method comprising the steps of:

   - (E60) obtaining pools $P_1,...,P_N$ of nucleotide sequences such that:

     ○ each pool $P_n$ containing nucleotide sequences of a DNA product $A_n$ obtained from genomic DNA samples $S_1,...,S_K$ of K population members;
     ○ each genomic DNA sample $S_k$ used to obtain a DNA product $A_n$ of a pool $P_n$ is also used to obtain only one DNA product $A_p$ of a pool $P_p$, the pools $P_n$ and $P_p$ being so-called concordant pools,
     ○ each pair of concordant pools $P_n$ and $P_p$ shares only one originating genomic DNA sample $S_{k*}$ from which the DNA products $A_n$ and $A_p$ are obtained,

   - (E70) identifying mutation(s) by clustering said nucleotide sequences of DNA products and aligning the clustered nucleotide sequences with a reference sequence,
   - (E80) Attributing a detection reliability score to each of the mutation(s) identified in the DNA products; and
   - (E90) detecting a mutation in a genome if the following conditions are satisfied:

     ○ the mutation is identified in the nucleotide sequences of DNA products only in a pair of concordant pools ; and
     ○ the detection reliability score associated with said mutation exceeds a reference value.

2. The method of claim 1, wherein the reference value is determined based on the detection reliability scores of mutations identified in the nucleotide sequences of non-concordant pools and identified in the nucleotide sequences of concordant pools.

3. The method of any of the preceding claims, wherein the reference value is within a range.

4. The method of any of the preceding claims, wherein a further condition for detecting (E90) said mutation in said genome is that : said mutation is identified (E70) at least a predetermined number of times, in particular two, in at least one pool of said pair of concordant pools.

5. The method of any of the preceding claims, wherein the fragmented DNA product is a fragmented DNA product of target sequences.

6. The method of any of the preceding claims, wherein the fragmented DNA product is an amplified fragmented DNA product.

7. The method of any of the preceding claims, wherein the number of genomic DNA samples K in a pool verifies $K=\sqrt{X}$ and the number of pools N verifies $N=2\cdot\sqrt{X}$ with X denoting the number of members in the population.

8. A method for detecting at least one mutation in the genome of a member of a population $M_1,..., M_X$, comprising the steps of:

a) providing genomic DNA samples $S_1,...,S_K$ of each population members;
b) pooling the genomic DNA samples obtained in step a) thereby obtaining a DNA product in each pool $P_n$, wherein each pools $P_1,...,P_N$ comprises K genomic DNA samples of K population members, wherein each of the K genomic DNA samples is present in only two pools $P_n$ and $P_p$, the pools $P_n$ and $P_p$ being so-called concordant pools, and wherein each pair of concordant pools $P_n$ and $P_p$ shares only one common genomic DNA sample ;
c) optionally, fragmenting the DNA product in each pool $P_n$, thereby obtaining a fragmented DNA product in each pool $P_n$ ;
d) optionally, in addition to step c), capturing a target sequence of interest in the fragmented DNA product in each pool $P_n$, thereby obtaining a captured DNA product in each pool $P_n$ ;
e) optionally, amplifying the DNA product of step b), the fragmented DNA product of step c) or the captured DNA product of step d) in each pool $P_n$, thereby obtaining an amplified DNA product in each pool $P_n$;
f) determining the nucleotide sequence of the DNA product of step b), of the fragmented DNA product of step c) or of the amplified DNA product of step e) in each pool $P_n$, thereby obtaining pools $P_1,...,P_N$ of nucleotide sequences ;
g) detecting a mutation by implementing the computer implemented method according to any of claims 1 to 6;
h) optionally, confirming a mutation that is detected in step g) by determining the sequence of a target sequence that is suspected to carry said mutation from a genomic DNA sample of the member that is suspected to carry said mutation.

9. The method according to claim 8, wherein steps c) to f) correspond to a Next-Generation Sequencing (NGS) process, such as Massively Parallel Signature Sequencing (MPSS) process, Illumina sequencing, Pyrosequencing, SOLiD sequencing process, Single molecule real time (RNAP) sequencing process or Single molecule SMRT sequencing process.

10. A computer (COMP) comprising a processor (5) and a memory (6), said memory comprising a program (PG) comprising instructions, which when executed by said processor implements a detection according to any one of Claims 1 to 7.

11. A computer program (PG) comprising instructions, which when executed by a processor (10) implements a detection according to any one of Claims 1 to 7.

12. A computer readable medium (6) comprising a computer program (PG) according to Claim 11.

E10 — Providing genomic DNA (gDNA) samples

E20 — Pooling the gDNA samples thereby obtaining a DNA product in each pool

E30 — Fragmenting the DNA product in each pool (Optional)

E40 — Capturing a target sequence of interest (Optional)

E50 — Amplifying the DNA product (Optional)

E60 — Determining the nucleotide sequence of the DNA product thereby obtaining pools of nucleotide sequences

E70 — Identifying mutations by clustering said nucleotide sequences and aligning the clustered nucleotide sequences with a reference sequence

E80 — Attributing a detection reliability score to each of the identified mutations

E90 — Detecting a mutation

# FIG. 1

**FIG. 2**

**FIG. 3**

☒ **Variants carried by 2 non-concordant pools**   ☐ **Variants carried by 2 concordant pools**

**FIG. 4**

FIG. 5

FIG. 6

Europäisches Patentamt
European Patent Office
Office européen des brevets

# EUROPEAN SEARCH REPORT

Application Number

EP 23 30 6874

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MARCO SAVARESE ET AL: "MotorPlex provides accurate variant detection across large muscle genes both in single myopathic patients and in pools of DNA samples", ACTA NEUROPATHOLOGICA COMMUNICATIONS, BIOMED CENTRAL LTD, LONDON, UK, vol. 2, no. 1, 11 September 2014 (2014-09-11), page 100, XP021198872, ISSN: 2051-5960, DOI: 10.1186/S40478-014-0100-3 * Title p.2,col.1,par.2 p.2,col.2,par.5 p.2,col.2,par.2 p.2,col.2,par.3 p.3,col.2,par.2 p.3,col.1,par.1 p.4,col.1,par.2 supplementary table 2 * | 1-12 | INV. G16B20/20 C12Q1/6869 G16B30/00 |
| Y | -& Savarese Marco ET AL: "MotorPlex provides accurate variant detection across large muscle genes both in single myopathic patients and in pools of DNA samples – supplementary Figure 2: Pooling strategy", Acta Neuropathol Commun, 11 September 2014 (2014-09-11), pages 1-1, XP093147512, DOI: 10.1186/s40478-014-0100-3 Retrieved from the Internet: URL:https://www.ncbi.nlm.nih.gov/pmc/articles/PMC4172906/ [retrieved on 2024-04-03] ----- -/-- | 1-12 | TECHNICAL FIELDS SEARCHED (IPC) G16B C12Q |

The present search report has been drawn up for all claims

| Place of search | Date of completion of the search | Examiner |
|---|---|---|
| Munich | 3 April 2024 | Eberhardt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

## EUROPEAN SEARCH REPORT

**Application Number**

**EP 23 30 6874**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NADINE NORTON ET AL: "Genome-wide Studies of Copy Number Variation and Exome Sequencing Identify Rare Variants inas a Cause of Dilated Cardiomyopathy", THE AMERICAN JOURNAL OF HUMAN GENETICS, AMERICAN SOCIETY OF HUMAN GENETICS , CHICAGO , IL, US, [Online] vol. 88, no. 3, 29 January 2011 (2011-01-29), pages 273-282, XP028178499, ISSN: 0002-9297, DOI: 10.1016/J.AJHG.2011.01.016 [retrieved on 2011-02-02] * p.274,col.1,par.2 * | 1-12 | |
| Y | US 2016/281166 A1 (BHATTACHARJEE ARINDAM [US] ET AL) 29 September 2016 (2016-09-29) * [0043] * | 1-12 | |
| Y | ERIC VILLARD ET AL: "A genome-wide association study identifies two loci associated with heart failure due to dilated cardiomyopathy", EUROPEAN HEART JOURNAL (ONLINE), OXFORD UNIVERSITY PRESS, GB, US, NL, [Online] vol. 32, no. 9, 1 April 2011 (2011-04-01), pages 1065-1076, XP002644271, ISSN: 1522-9645, DOI: 10.1093/EURHEARTJ/EHR105 [retrieved on 2011-04-01] * p.1067, col.1,par.1 * | 1-12 | TECHNICAL FIELDS SEARCHED (IPC) |

The present search report has been drawn up for all claims

| Place of search | Date of completion of the search | Examiner |
|---|---|---|
| Munich | 3 April 2024 | Eberhardt, Anja |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

**EP 23 30 6874**

This annex lists the patent family members relating to the patent documents cited in the above-mentioned European search report.
The members are as contained in the European Patent Office EDP file on
The European Patent Office is in no way liable for these particulars which are merely given for the purpose of information.

**03-04-2024**

| Patent document cited in search report | Publication date | Patent family member(s) | Publication date |
|---|---|---|---|
| US 2016281166 A1 | 29-09-2016 | NONE | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **PINTO, Y. M. et al.** Proposal for a revised definition of dilated cardiomyopathy. *Eur. Heart J.*, 2016, vol. 37, 1850-1858
- **CAO, C.; SUN, X.** Combinatorial pooled sequencing: experiment design and decoding. *Quant. Biol.*, 2016, vol. 4, 36-46
- **ZUZARTE, P. C. et al.** A Two-Dimensional Pooling Strategy for Rare Variant Detection on Next-Generation Sequencing Platforms. *PLoS ONE*, 2014, vol. 9, e93455
- **HAAS, J. et al.** Atlas of the clinical genetics of human dilated cardiomyopathy. *Eur. Heart J.*, 2015, vol. 36, 1123-1135
- **JORDAN, E. et al.** Evidence-Based Assessment of Genes in Dilated Cardiomyopathy. *Circulation*, 2021, vol. 144, 7-19
- **HARTMAN, P. et al.** Next generation sequencing for clinical diagnostics: Five year experience of an academic laboratory. *Mol. Genet. Metab. Rep.*, 2019, vol. 19, 100464
- **HERSHBERGER, R. E. et al.** Genetic evaluation of cardiomyopathy: a clinical practice resource of the American College of Medical Genetics and Genomics (ACMG). *Genet. Med.*, 2018, vol. 20, 899-909
- **HERMAN, D. S. et al.** Truncations of Titin Causing Dilated Cardiomyopathy. *N. Engl. J. Med.*, 2012, vol. 366, 619-628
- **ROBERTS, A. M. et al.** Integrated allelic, transcriptional, and phenomic dissection of the cardiac effects of titin truncations in health and disease. *Sci. Transl. Med.*, 2015, vol. 7
- **STOLFO, D.; COLLINI, V.; SINAGRA, G.** Advanced Heart Failure in Special Population: Cardiomyopathies and Myocarditis. *Heart Failures Clinics*, 2021, vol. 17 (4), 661-672